# EUROPEAN PATENT APPLICATION

(11) **EP 4 353 742 A1**
(43) Date of publication of application: **17.04.2024**
(21) Application number: 22820595.1
(22) Date of filing: 10.06.2022
(51) Int. Cl.: C07K 14/50, A61K 38/00, A61P 3/00, A61P 9/00

(54) **DEVELOPMENT OF NOVEL FGF21 VARIANT, AND PRODUCTION TECHNIQUE AND USE THEREOF**

(30) Priority: 10.06.2021 KR 20210075460
(71) Applicant: Todd Pharm Co., Ltd., Seodaem un-gu, Seoul 03760 (KR)
(72) Inventor: CHA, Sun Shin, Seoul 03709 (KR); JUNG, Ye-Eun, Seoul 03760 (KR); BAE, Da-Woon, Seoul 04422 (KR); JEONG, Bo-Gyeong, Seoul 04114 (KR); JUNG, Yeon Ju, Seoul 03778 (KR); KANG, Bu-Gyeong, Seoul 03767 (KR); KIM, Myeong-Yeon, Seoul 03704 (KR); YOUN, So Yeon, Seoul 03721 (KR); LEE, Jung-Hyun, Busan 48222 (KR); YIM, Hyung-Soon, Seoul 03748 (KR); LEE, Kyeong Won, Busan 49057 (KR); AN, Young Jun, Ansan-si, Gyeonggi-do 15629 (KR); KWON, Kae Kyoung, Busan 48723 (KR); KANG, Sung Gyun, Busan 48075 (KR); LEE, Hyun Sook, Busan 48075 (KR)
(74) Representative: Brevalex
(86) International application number: PCT/KR2022/008202
(87) International publication number: WO 2022/260468

(57) **Abstract**

The present disclosure relates to the development of novel FGF21 variants and methods of production or purification thereof. A novel FGF21 variant according to an embodiment of the present disclosure exhibits an approximately 6-fold improved potency in signal transduction activation compared to the wild type, and the variant may be evaluated as a valuable drug candidate as a biobetter.

## Description

### Technical Field

This application claims priority to Korean Patent Application No. 10-2021-0075460, filed on June 10, 2021, which is hereby incorporated by reference in its entirety.

This relates to the development of novel FGF21 variants and methods of production or purification of the same.

### Background Art

Mammalian-derived fibroblast growth factor (FGF) is a protein that regulates glucose/lipid metabolism, cell differentiation and proliferation, etc. by binding to the ectodomain of a receptor present in the cell membrane and activating the tyrosine kinase domain of the receptor present in the cytoplasm. The FGF family consists of 22 structurally similar proteins and is divided into 7 subfamilies.

Among them, FGFs belonging to five subfamilies (FGF1, FGF4, FGF7, FGF8, and FGF9 subfamilies) require heparin or heparan sulfate when binding to receptors. Heparin/heparan sulfate, which exists on the cell surface or extracellular matrix, is a polysaccharide with a (-) charge belonging to the glycosaminoglycan (GAG) family and regulates its function through interactions with various proteins. FGFs, which have high binding affinity to heparin/heparan sulfate, function as a paracrine by acting near the cells that secrete them and exerting their physiological activity.

FGF21 is a protein encoded by an FGF21 gene in mammals and belongs to an endocrine subfamily that includes FGF23 and FGF15/19. FGF21 is a major endogenous agonist of FGF receptor 1c and its co-receptor β-Klotho.

FGF21 has recently been widely studied as an active ingredient in various therapeutics. Specifically, in July 2019, Yuhan Corporation signed a licensing agreement with Boehringer Ingelheim International GmbH for the purpose of developing a therapeutic for nonalcoholic steatohepatitis (NASH), in which the drug candidate is designed as a dual agonist in which FGF21 and Glucagon-like peptide-1 (GLP-1) are fused. Currently, it is known that non-clinical toxicity testing studies have been completed for the drug candidate and joint research is underway with the goal of entering clinical trials. In addition, Merck & Co., Inc. is also developing MK-3655, a monoclonal antibody that recognizes the β-Klotho/FGFR1c complex for the purpose of treating NASH, and it may be seen that FGF21 is a new drug candidate that is attracting attention in domestic and international pharmaceutical industry markets as a therapeutic for diabetes and NASH.

In addition, various pharmacological effects of FGF21 administration on brain, bone, pancreas, liver, etc. are being studied, and the scope of application of therapeutics with FGF21 as an active ingredient is expected to gradually expand.

Accordingly, the inventors of the present disclosure have made diligent efforts to develop FGF21 variants with improved activity, and as a result, have developed a variant with approximately 6-fold improved potency in signal transduction activation compared to the wild type, thereby completing the present disclosure.

### Disclosure

### Technical Problem

One aspect relates to novel FGF21 variants and methods for producing the same.

However, the problem to be solved herein is not limited to the problem described above.

Other problems not mentioned may be apparent to those skilled in the art from the following description.

### Technical Solution

In one aspect, provided is a polypeptide including FGF21 variants, wherein the variant is a polypeptide which is featured by one or more mutations in the FGF21 wild type.

In another aspect, provided is a method of producing FGF21 variants, including processes of culturing transgenic organisms producing the FGF21 variants; and heating the transgenic organisms, cultures, or media at a temperature of 40 °C or above.

In another aspect, provided is a method of purifying FGF21 variants, including a process of heating a mixture including the FGF21 variants and impurities at a temperature of 40 °C or above.

In another aspect, provided is an FGF21 variant prepared by the method above.

In another aspect, provided is a pharmaceutical composition for treating a metabolic disorder or cardiovascular disorder including the FGF21 variants.

In another aspect, provided is a method of treating a metabolic disorder or cardiovascular disorder, including a process of administering the pharmaceutical composition to a subject.

In another aspect, provided is a use of the pharmaceutical composition for treating a metabolic disorder or cardiovascular disorder.

### Advantageous Effects

A novel FGF21 variant according to an embodiment exhibits an approximately 6-fold improved potency in signal transduction activation compared to the wild type, and the variant may be evaluated as a valuable drug candidate as a biobetter.

### Description of Drawings

FIG. 1 is a diagram showing the results of sequence comparison analysis between hFGF21 and wFGF21, wherein shading indicates sequences with specific differences in the C-terminal tails of the two sequences.
FIG. 2 is a diagram showing the results of structural analysis for the X-ray crystal structure of the hFGF21CT/β-Klotho complex (PDB code: 5VAQ), herein enlarged residues correspond to the mutation sites of the FGF21 variants for design strategies ① and ②.
FIG. 3 is a diagram showing the sequences of the wild type FGF21 and its variant.
FIG. 4 is a diagram showing (left) the result of SDS-PAGE analysis before and after heat-treatment of a supernatant overexpressing a Trx-FGF21 variant and (right) the result of SDS-PAGE analysis for the finally purified FGF21 variant ③.
FIG. 5 is a diagram showing data comparing the activities between commercially available FGF21 (wild type) and FGF21 N4 purified by heat-treatment.
FIG. 6 is a diagram showing in vitro cell assay of the wild type FGF21 and its variants ①, ②, and ③ on 3T3L1 adipocytes.
FIG. 7 is a diagram showing data on FGFR1c activation experiments at the indicated concentrations of the wild type FGF21 and its variants ①, ②, and ③.

### Best Mode

Embodiments of the present disclosure are described in detail below with reference to the accompanying drawings to facilitate practice by one of ordinary skill in the art to which the present disclosure belongs. However, the present disclosure may be implemented in various other forms and is not limited to the embodiments described herein. In order to clearly illustrate the present disclosure in the drawings, parts that are not related to the description are omitted, and similar parts are given similar reference numerals throughout the specification.

Throughout the present disclosure, when a part is said to be "connected" to another part, this includes not only cases where it is "directly connected," but also cases where it is "indirectly connected" with substances such as other linkers, etc. in between.

Throughout the present disclosure, when a part is said to "include" a component, refers to that it may further include other components, not exclusive of other components, unless specifically stated to the contrary. As used throughout the present disclosure, the terms "about," "substantially," etc. are intended to mean at or near the numerical value when manufacturing and material tolerances inherent in the stated value are given, and are used for the purpose of clarity and to prevent unconscionable infringers from taking unfair advantage of the present disclosure where precise or absolute numerical values are stated. As used throughout the present disclosure, the terms "processes to" or "processes of" do not mean "processes for".

Throughout the present disclosure, the term "combination(s) thereof' as included in a Markush-style representation refers to one or more mixtures or combinations selected from the group of components set forth in the Markush-style representation, including one or more selected from the group of components.

Throughout the present disclosure, references to "A and/or B" shall mean "A or B, or A and B".

Hereinafter, embodiments and examples of the present disclosure are described in detail with reference to the accompanying drawings. However, the present disclosure may not be limited to these embodiments and examples and the drawings.

In one aspect, provided is a polypeptide including an FGF21 variant, wherein the variant is a polypeptide which features including one or more mutations in a wild type FGF21.

As used throughout the present disclosure, the term "fibroblast growth factor 21 (FGF21)" refers to a protein of 209 amino acids whose mature form (29-209aa), in which a signal peptide (1-28aa) has been removed, has a lower binding affinity for heparin/heparan sulfate than paracrine FGF proteins, thus able to travel in the blood away from the cells where it is secreted. Due to these properties, FGF21 functions as an endocrine hormone, reaching distant targets and exerting physiological activity. FGF21 acts simultaneously in a complex with FGFR1c, one of FGF receptors present on the cell surface, and a co-receptor β-Klotho. While heparin/heparan sulfate is present in all cells, β-Klotho is mainly distributed in adipose tissue, liver, and pancreas. Therefore, FGF21 regulates glucose and lipid metabolism by affecting the activity of cells expressing FGFR1c and β-Klotho. For example, FGF21 has been reported to be effective in improving metabolic diseases such as type 2 diabetes and fatty liver disease, and in particular, FGF21 has emerged as an important drug candidate for the development of therapeutics for lipid metabolism disorders such as nonalcoholic fatty liver disease and nonalcoholic steatohepatitis.

As used throughout the present disclosure, the term "FGF21 variant" refers to a variant including a change in form of the amino acid sequence of wild type FGF21 in which one or more amino acids are substituted, deleted, or inserted. As used herein, "FGF21 variant" may be used interchangeably with "FGF variant protein".

In an embodiment, the polypeptide may include an FGF21 variant, and may specifically consist of an FGF21 variant.

In an embodiment, the wild type FGF21 may be a mature form in which the signal peptide has been removed, and specifically, may consist of amino acids 1^{st} to 28^{th}, which are the signal peptide, removed from the FGF21 protein consisting SEQ ID NO: 1, and consisting of amino acids 29^{th} to 209^{th}, and specifically, may include amino acids of SEQ ID NO: 2.

In an embodiment, the FGF21 variant may have 1 to 4 amino acids from the N-terminus deleted.

In an embodiment, the FGF21 variant may be one in which one or more amino acids selected from the group consisting of Q184, S195, P199, S200, Q201, and A208 substituted with another amino acid, specifically may be substituted with one of 20 amino acids (Q, E, G, A, S, T, C, V, L, I, M, P, F, Y, W, D, N, H, K, and R).

In an embodiment, the FGF21 variant may be substituted with which one or more amino acids selected from the group consisting of Q184E, S195G, P199G, S200G, Q201H, and A208T, specifically may be substituted with S195G, P199G, S200G, Q201H, and A208T.

In an embodiment, an S195G substitution may be a substitution of the 167^{th} amino acid of SEQ ID NO: 2 (serine) with glycine, and a P199G substitution may be a substitution of the 171^{st} amino acid of SEQ ID NO: 2 (proline) with glycine, S200G substitution may be a substitution of the 172^{nd} amino acid of SEQ ID NO: 2 (serine) with glycine, Q201H substitution may be a substitution of the 173^{rd} amino acid of SEQ ID NO: 2 (glutamine) with histidine, and A208T substitution may be a substitution of the 180^{th} amino acid of SEQ ID NO: 2 (alanine) with threonine.

In an embodiment, the FGF21 variant may be one of the amino acid sequences of SEQ ID NO: 3 to 6.

In an embodiment, the FGF21 variant may have improved activity compared to the wild type, specifically, may have improved about 6-fold potency in signal transduction activation compared to the wild type.

In another aspect, provided is a method of producing FGF21 variants, including processes of culturing transgenic organisms producing the FGF21 variants; and heating the transgenic organisms, cultures, or media at a temperature of 40 °C or above. The repetitive description of the polypeptide including the FGF21 variants applies equally to the method of producing the FGF21 variants.

In an embodiment, the FGF21 variants may include a polypeptide including one or more of the amino acid sequences of SEQ ID NO: 3 to 6, and specifically may be including the amino acid sequence of SEQ ID NO: 6.

In an embodiment, wild type FGF21 including a β-trefoil core structure consisting of amino acid sequences 41^{st} to 173^{rd} of FGF21, as well as various variants predicted to include the β-trefoil core structure, did not denature or precipitate at temperatures from 40 °C to 100 °C or above, whereas, it may be seen that upon re-cooling after heat-treatment, the original protein structure was retained, and upon such re-cooling, the physiological activity of FGF21 was also retained, indicating its high heat-stability/heat-elasticity.

In an embodiment, the transgenic organism may include one of polynucleotides encoding FGF21 variants, and specifically may include a gene construct or recombinant vector harboring a gene encoding the FGF21 variant.

In an embodiment, the transgenic organism producing one of the FGF21 variants may include a gene construct or recombinant vector harboring a gene encoding the FGF21 variant, thereby expressing it.

As used throughout the present disclosure, the term "recombinant vector" refers to a vector which may express a peptide or protein encoded by a heterologous nucleic acid inserted into the vector, desirably a vector prepared to include a polynucleotide encoding the FGF21. The "vector" refers to any vehicle for the introduction and/or transfer of a base into a host cell in a test-tube, in vitro, or in vivo, and may be a replicon to which another DNA fragment may bind, resulting in the replication of the bound fragment, and "replicative unit" refers to any genetic unit (for example, plasmid, phage, cosmid, chromosome, virus, etc.) that functions as an autonomous unit of DNA replication in vivo, in other words, is capable of replication under its own control.

The recombinant vector or recombinant expression vector may desirably include a promoter, which is a transcription initiation factor to which RNA polymerase binds, any operator sequence for regulating transcription, a sequence encoding a suitable mRNA ribosome binding site and a sequence regulating termination of transcription and translation, a terminator, etc., more desirably, may additionally include a gene in the 5' UTR region of M17 to increase the amount of protein synthesis and an HDEL gene to minimize protein degradation so that the protein may be stably maintained in the endoplasmic reticulum, etc., and more desirably, may additionally include a tag gene for increasing the production of recombinant protein, a tag gene for maintaining the structural stability of the recombinant protein, a tag gene for easily separating the recombinant protein, and a selection marker gene such as an antibiotic resistance gene for selecting transgenic organism, etc., tag for easy isolation may typically include Avi tag, Calmodulin tag, polyglutamate tag, E tag, FLAG tag, HA tag, His tag, Myc tag, S tag, SBP tag, IgG-Fc tag, CTB tag, Softag 1 tag, Softag 3 tag, Strep tag, TC tag, V5 tag, VSV tag, Xpress tag, etc., the marker gene for screening may typically include a herbicide resistance gene such as glyphosate or phosphinothricin, an antibiotic resistance gene such as kanamycin, G418, bleomycin, hygromycin, chloramphenicol, and aadA gene, etc., the promoter may typically include a pEMU promoter, MAS promoter, histone promoter, Clp promoter, cauliflower mosaic virus-derived 35S promoter, cauliflower mosaic virus-derived 19S RNA promoter, plant actin protein promoter, ubiquitin protein promoter, cytomegalovirus (CMV) promoter, simian virus 40 (SV40) promoter, respiratory syncytial virus (RSV) promoter, elongation factor-1 alpha (EF-1α) promoter, etc., the terminator is typically nopaline synthase (NOS), rice amylase RAmy1 A terminator, phaseolin terminator, terminator of the octopine gene of *Agrobacterium tumefaciens,* rrnB1/B2 terminator of *Escherichia coli,* etc., there is no limit to the type of the inserted gene as long as it is a type that is already used in the production of recombinant protein.

As used throughout the present disclosure, the term "transformation" refers to an alteration of the genetic properties of an organism by injected DNA, and "transgenic organism" refers to an organism prepared by injecting a foreign gene by molecular genetic methods, desirably an organism transformed by the recombinant expression vector of the present disclosure, the organism may be any living organism such as microorganisms, eukaryotic cells, insects, animals, plants, etc., desirably, but not limited to, *Escherichia coli, Salmonella, Bacillus,* yeast, animal cells, mice, rats, dogs, monkeys, pigs, horses, cattle, *Agrobacterium tumefaciens,* plants, etc. The transgenic organisms may be prepared by methods such as transformation, transfection, agrobacterium-mediated transformation, particle gun bombardment, sonication, electroporation, polyethylene glycol (PEG)-mediated transformation, etc., but not limited to, as long as the vector may be injected.

In an embodiment, the FGF21 variant may have heat-stability or heat-elasticity.

As used throughout the present disclosure, the term "heat-stability" refers to the property that, under heat-treatment conditions, the protein does not denature, precipitate, etc., and its original feature/function remain the same/similar to those before the heat-treatment, such as the protein's bioactive function is not degraded or changed, despite the application of heat of a certain level or more.

According to an embodiment, when the FGF21 variant is heat-treated, it may be confirmed that denaturation and precipitation do not occur and the bioactive function, such as FGFRIc receptor activation, etc., is maintained despite heat-treatment at a temperature of 100 °C or above, indicating that the FGF21 protein has a heat-stable property.

As used throughout the present disclosure, the term "heat-elasticity" refers to a protein recovering the same/similar property/function upon re-cooling after a certain level or more of heat is applied under heat-treatment conditions.

In an embodiment, the FGF21 variant may have a β-trefoil structure.

As used throughout the present disclosure, the term "β-trefoil (or "β-trefoil fold") structure refers to a protein folding structure consisting of six β hairpins, each formed from two β strands. The structure forms a β-barrel with a triangular "cap" consisting of three hairpins, wherein the structure has an approximate threefold symmetric structure.

In an embodiment, the method may include a process of heating the transgenic organism, culture, or media to a temperature above the temperature at which a typical protein may be denatured, and specifically, the temperature may be 40 °C or above, 45 °C or above, 50 °C or above, 55 °C or above, 60 °C or above, 65 °C or above, 70 °C or above, 75 °C or above, 80 °C or above, 85 °C or above, 90 °C or above, 95 °C or above, or 100 °C, and more specifically, 40 °C or above to 300 °C or below, 40 °C or above to 250 °C or below, 40 °C or above to 200 °C or below, 40 °C or above to 150 °C or below, 40 °C or above to 120 °C or below, 50 °C or above to 300 °C or below, 50 °C or above to 250 °C or below, 50 °C or above to 200 °C or below, 50 °C or above to 150 °C or below, 50 °C or above to 120 °C or below, 60 °C or above to 300 °C or below, 60 °C or above to 250 °C or below, 60 °C or above to 200 °C or below, 60 °C or above to 150 °C or below, 60 °C or above to 120 °C or below, 70 °C or above to 300 °C or below, 70 °C or above to 250 °C or below, 70 °C or above to 200 °C or below, 70 °C or above to 150 °C or below, 70 °C or above to 120 °C or below, 80 °C or above to 300 °C or below, 80 °C or above to 250 °C or below, 80 °C or above to 200 °C or below, 80 °C or above to 150 °C or below, 80 °C or above to 120 °C or below, 90 °C or above to 300 °C or below, 90 °C or above to 250 °C or below, 90 °C or above to 200 °C or below, 90 °C or above to 150 °C or below, 90 °C or above to 120 °C or below, but not limited thereto. In an embodiment, the FGF21 variant may be one in which a heat-stable protein tag is additionally bound.

In an embodiment, the heat-stable protein tag may be connected to the N-terminus or C-terminus of the FGF21 variant, but is not limited thereto as long as the protein production/purification efficiency may be improved without affecting the feature/function, etc. of the FGF21 variant.

In an embodiment, the heat-stable protein tag is a heat-resistant protein tag that does not denature upon application of heat, and may be one or more selected from the group consisting of heat-stable proteins such as a thermophilic organism-derived protein, thioredoxin (Trx), and DnaK, etc.

In an embodiment, the FGF21 variant and the protein tag may be directly connected or connected via a linker. The linker may consist of, for example, a nucleic acid or peptide, etc. but is not particularly limited thereto, as long as it does not affect the property/function, etc. of the fusion protein to which the FGF21 variant or protein tag is connected to.

In an embodiment, the linker may be cleaved or degraded by various biological or chemical actions, such as enzymatic reaction, in intracellular or extracellular processes. Specifically, the linker may be cleaved by protease treatment using the Tobacco etch virus (TEV) protease or a specific sequence recognized by protease such as Thrombin, Factor Xa, etc. as the linker, or may be cleaved by heat-treatment (40 °C to 42 °C, 3 hours) with hydroxylamine (NH₂OH) using a linker including asparagine-glycine (Asn-Gly).

In an embodiment, a process of culturing the transgenic organism is not particularly limited thereto, but may be performed by a known batch culture method, continuous culture method, fed-batch culture method, etc. The culture conditions may include, but not limited to, the use of a basic compound (for example: sodium hydroxide, potassium hydroxide, or ammonia) or an acidic compound (for example, phosphoric acid or sulfuric acid) to adjust the appropriate pH (for example: pH 5 to 9, specifically pH 6 to 8, most specifically pH 6.8), and the introduction of oxygen or an oxygen-containing gas mixture into the culture may maintain an aerobic condition. The culture temperature may be maintained at 20 °C to 45 °C, specifically 25 °C to 40 °C, and the culture may be incubated for about 10 hours to 160 hours. The FGF21 variant produced by the above culture may be secreted into the medium or retained in the transgenic organisms.

Furthermore, the culture medium used may be, but not limited to, sugar and carbohydrate (for example: glucose, sucrose, lactose, fructose, maltose, molasses, starch, and cellulose) as a carbon source, oil and fat (for example: soybean oil, sunflower seed oil, peanut oil, and coconut oil), fatty acid (for example: palmitic acid, stearic acid, and linoleic acid), alcohol (for example: glycerol and ethanol), and organic acid (for example: acetic acid), etc. used individually or in combination. A nitrogen source may be, but not limited to, a nitrogen-containing organic compound (for example: peptone, yeast extract, wort, malt extract, corn soak, soybean meal, and urea), or inorganic compound (for example: ammonium sulfate, ammonium chloride, ammonium phosphate, ammonium carbonate, and ammonium nitrate), etc. used individually or in combination. A phosphorus source may be, but not limited to, dipotassium dihydrogen phosphate, dipotassium hydrogen phosphate, and the corresponding sodium-containing salt, etc. used individually or in combination, and may include other metal salt (for example: magnesium sulfate or iron sulfate), essential growth-promoting substance such as amino acid and vitamins.

In an embodiment, the method may further include a process of removing a denatured protein by heat-treatment.

In an embodiment, the process of removing the denatured protein may include precipitating the heat-treated denatured protein, specifically the protein derived from the transgenic organism, by centrifugation, etc., and then removing the precipitate. With the process, the FGF21 variant that has not been denatured by heat-treatment is present in the supernatant portion after centrifugation and may be readily obtained.

In an embodiment, the method may additionally include a process of recovering the produced FGF21 variant. This recovery process may be a process of recovery from cultured cells or supernatant thereof, and a procedure suitable for recovery may be selected by those skilled in the art. The method of recovering the FGF21 variant produced in the above culture process may be accomplished by collecting the desired product from the culture using any suitable method known in the art, depending on the culture method, for example, batch, continuous, fed-batch culture method, etc.

In an embodiment, a process of recovering the FGF21 variant may be removing proteins (protein derived from the transgenic organism) excluding the FGF21 variant precipitated by denaturation by heat-treatment, for example by centrifugation, etc., and obtaining only the FGF21 variant that has not been denatured by heat-treatment due to its heat-stability (heat-elasticity).

Provided is a method of purifying FGF21 variants, including a process of heating a mixture including the FGF21 variant and impurities to a temperature of 40 °C or above.

In an embodiment, the impurities may include a protein or the like other than the FGF21 variant arising from a process of producing the FGF21 variant, specifically including protein and the like derived from a transgenic organism producing the FGF21 variant.

In an embodiment, a process of purifying FGF21 variants may be to remove impurities (proteins derived from the transgenic organism) that have precipitated as a result of being denatured by heat-treatment, for example by centrifugation, etc. and to obtain only the FGF21 variant that has not been denatured due to its heat-stability (heat-elasticity) despite heat-treatment.

Another aspect provides a novel FGF21 variant.

Another aspect provides a composition for treating a metabolic disorder or cardiovascular disorder including a novel FGF21 variant.

In an embodiment, the metabolic disorder or cardiovascular disorder may be selected from hypercholesterolemia, dyslipidemia, hypertriglyceridemia, nonalcoholic fatty liver disease (NAFLD), nonalcoholic steatohepatitis (NASH), type 2 diabetes, and obesity.

In an embodiment, treating, preventing, or managing the metabolic disorder or cardiovascular disorder may include reducing one or more of a subject's weight, liver fat content, elevated LDL-C, total-C, triglycerides, and Apo B levels.

In an embodiment, the metabolic disorder or cardiovascular disorder may be dyslipidemia, optionally mixed dyslipidemia, hypertriglyceridemia, optionally severe hypertriglyceridemia, or hypercholesterolemia, optionally primary hypercholesterolemia.

In an embodiment, the composition may be one or more of a pharmaceutical composition, a functional food composition, and a feed composition.

The pharmaceutical composition may additionally include a suitable carrier, an excipient, or a diluent generally used in the preparation of a pharmaceutical composition. Compositions including a pharmaceutically acceptable carrier may be in a variety of oral or parenteral formulations. When formulated, the composition may be prepared using commonly used diluents or excipients such as a filler, expander, binder, lubricant, disintegrating agent, surfactant, etc. Solid formulations for oral administration may include a tablet, powder, granule, capsule, etc., which may be formulated by mixing one or more compounds with at least one or more excipients, for example starch, calcium carbonate, sucrose or lactose, gelatin, etc.

Additionally, in addition to simple excipients, lubricants such as magnesium stearate, talc, etc. may also be used. Liquid formulations for oral administration include a suspension, oral liquid, emulsion, syrup, etc. which may contain a variety of excipients, for example a lubricant, sweetener, scent, and preservative, etc., in addition to commonly used simple diluents such as water and liquid paraffin. Formulations for parenteral administration may include a sterile aqueous solution, non-aqueous solvent, suspension, emulsion, lyophilizate, and suppository. Non-aqueous solvents and suspension solvent may include propylene glycol, polyethylene glycol, and vegetable oil such as olive oil, etc. As a base for the suppository, witepsol, macrogol, tween 61, cacao paper, laurin paper, glycerogelatin, etc. may be used.

In addition, the pharmaceutical composition may have any one formulation selected from the group consisting of a tablet, pill, powder, granule, capsule, suspension, oral liquid, emulsion, syrup, sterilized aqueous solution, non-aqueous solvent, suspension, emulsion, lyophilizate, and suppository.

In another aspect, provided is a method of treating a metabolic disorder or cardiovascular disorder, including a process of administering the pharmaceutical composition to a subject.

The pharmaceutical composition is administered in a pharmaceutically effective amount.

As used throughout the present disclosure, the term "administer" refers to introducing a pharmaceutical composition of the present disclosure into a subject by any suitable method, and the route of administration may be by a variety of routes, oral or parenteral, as long as it may reach the target tissue.

The pharmaceutical compositions of the present disclosure may be administered to an individual according to usual methods, routes of administration, and dosages used in the art, as appropriate for the purpose or need. Examples of routes of administration may be oral, parenteral, subcutaneous, intraperitoneal, intrapulmonary, and intranasal, and parenteral administration includes intramuscular, intravenous, intra-arterial, intraperitoneal, or subcutaneous administration.

In addition, appropriate dosage and number of doses may be selected according to methods known in the art, and the amount of pharmaceutical composition of the present disclosure actually administered and the number of doses may be appropriately determined by a variety of factors, such as the type of symptom to be treated, the route of administration, gender, health status, diet, age and weight of the individual, and severity of the disease.

As used herein, the term "pharmaceutically effective amount" refers to an amount sufficient to inhibit or alleviate a metabolic disorder or cardiovascular disorder at a reasonable benefit/risk ratio applicable to the medical use, and the effective dose level may be determined based on factors including the type and severity of the condition, age, gender, activity of the drug, sensitivity to the drug, time of administration, route of administration and elimination rate, duration of treatment, concomitant medications, and other factors well known in the medical field.

As used throughout the present disclosure, the term "subject" refers to any animal, including a human, which has or may develop a metabolic disorder or cardiovascular disorder, and by administering a pharmaceutical composition of the present disclosure to the subject, the metabolic disorder or cardiovascular disorder may be effectively treated.

In another aspect, provided is a use of the pharmaceutical composition for treating a metabolic disorder or cardiovascular disorder.

The functional food composition may be used by adding the active ingredients directly to food or with other foods or food ingredients, and may be used appropriately according to usual methods. The functional food compositions are not particularly restricted in terms of other ingredients other than containing the active ingredient as an essential ingredient in the proportions indicated, and may contain various flavoring agents or natural carbohydrates, etc., as additional ingredients, such as general beverages. Examples of the natural carbohydrate mentioned above are monosaccharides, for example, glucose, fructose, etc.; disaccharides, for example, maltose, sucrose, etc.; and polysaccharides, for example, dextrin, cyclodextrin, etc. general sugar, and sugar alcohol such as xylitol, sorbitol, erythritol, etc. As flavoring agents other than those described above, natural flavoring agents (such as thaumatin, stevia extract (for example rebaudioside A, glycyrrhizin, etc.)) and synthetic flavoring agents (such as saccharin, aspartame, etc.) may be advantageously used. The proportions of the natural carbohydrates may be suitably determined by the choice of those skilled in the art.

In addition to the above, the functional food composition may contain various nutrients, vitamins, minerals (electrolytes), flavoring agents such as synthetic flavoring agents and natural flavoring agents, etc., colorants and binders (cheese, chocolate, etc.), pectic acid and its salts, alginic acid and its salts, organic acids, protective colloidal thickeners, pH adjusters, stabilizers, preservatives, glycerin, alcohol, carbonation agents used in carbonated beverages, etc. These ingredients may be used independently or in combination. The proportions of these additives may also be appropriately selected by those skilled in the art.

Hereinafter, the present disclosure will be described in more detail with reference to the following embodiments, but the following embodiments are for illustrative purposes only, and the content of the present disclosure is not limited to the following embodiments.

### Example

### Example 1: Design and discovery of novel FGF21 variants

Various pharmaceutical companies around the world pay attention to FGF21 as a drug candidate for obesity, diabetes, and nonalcoholic steatohepatitis (hereinafter, NASH). FGF21 is a protein that regulates glucose and lipid metabolism by affecting cell activity in the brain, liver, pancreas, muscle, adipose tissue, etc. It forms a ternary complex with both FGFR1c and β-Klotho present on the cell surface of target organs and activates the tyrosine kinase domain, the cytosolic domain of FGFR1c, to trigger the signal transductions of intracellular kinases.

The FGF21 protein consists of 209 amino acids, and a novel variant was designed based on the mature form (29-209aa) in which the signal peptide (1-28aa) is removed and four amino acids at the N-terminus are removed, in order to develop a novel FGF21 variant, an FGF21 variant was designed using the following strategy.

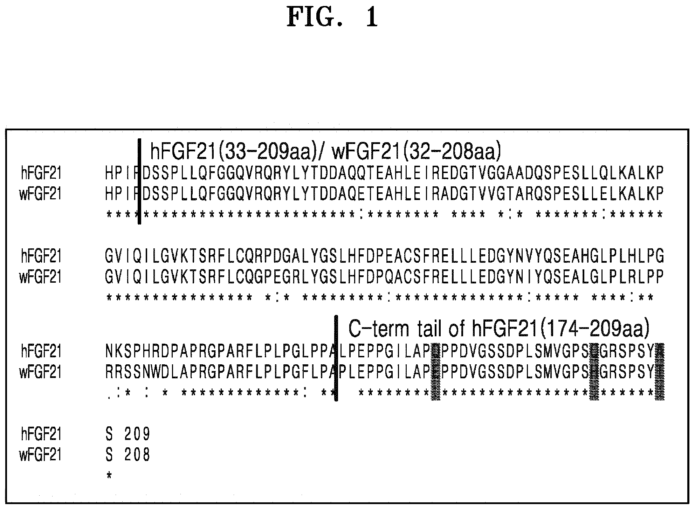

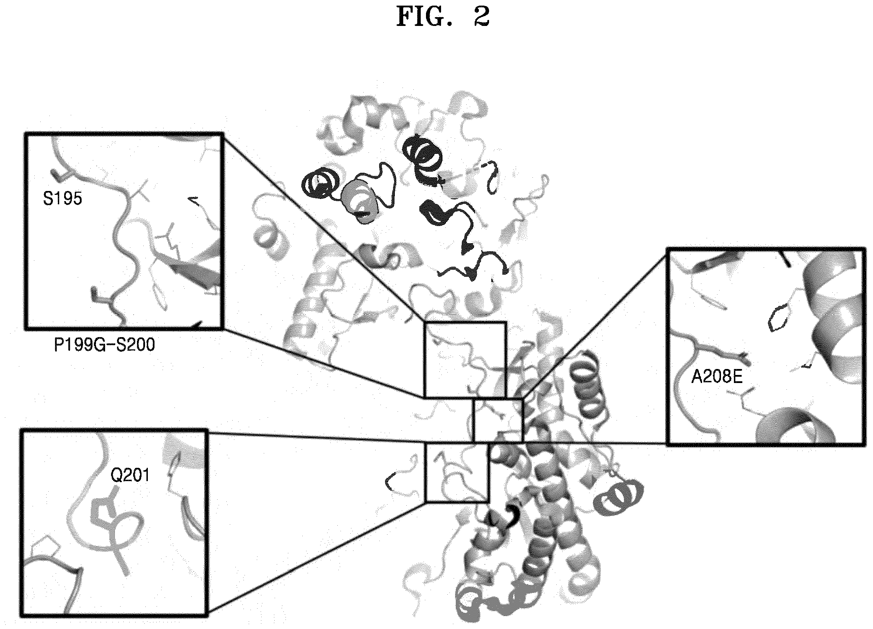

As a first strategy, the ortholog comparison technique was applied. Specifically, the sequence of north pacific minke whale-derived FGF21 (wFGF21) was selected through various mammalian gene analyses and comparison analysis with the humanderived FGF21 (hFGF21) sequence. As a result, it was confirmed that there were specific differences in the three residues (Gln184 & Gln201 & Ala208) shaded in FIG. 1. In addition, the crystal structure (PDB code: 5VAQ) of the hFGF21CT (186-209aa) complexed with β-Klotho was analyzed. As a result, it was determined that the interaction between hFGF21CT and β-Klotho would be strengthened when Gln201 and Ala208 of hFGF21 were replaced with the corresponding residues His200 and Thr207 of wFGF21 as illustrated in FIG. 2. Therefore, mutation ① (Gln184Glu & Gln201His & Ala208Thr) was developed in which three residues of hFGF21CT were substituted with residues of wFGF21CT.

As a second strategy, the engineering sites were selected by analyzing the crystal structure of the hFGF21CT/β-Klotho complex. Based on structural analysis, it was possible to identify three regions (Ser195 & Pro199 & Ser200) in the sequence of FGF21CT where residues do not participate in the interaction with β-Klotho. Therefore, for the purpose of enhancing the activity and improving the stability of FGF21, mutation ② (Ser195Gly & Pro199Gly & Ser200Gly) was developed in which glycine was introduced. This is a structure-based engineering strategy to enhance interactions with β-Klotho and simultaneously to increase resistance against protease attack.

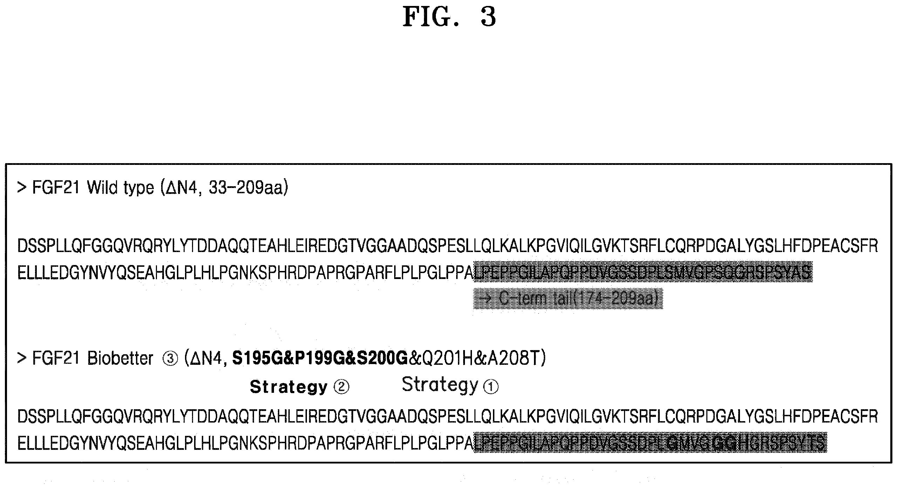

Through the above two strategies, a synergistic effect may be expected when variants ① and ② are combined, so FGF21 variant ③, which combines the two strategies, was developed as a novel FGF21 variant (FIG. 3).

### Example 2: Production and purification of FGF21 variants

In order to produce and purify the FGF21 variant designed in Example 1 above, a purification technique using heat-elasticity was applied based on the results of previous studies.

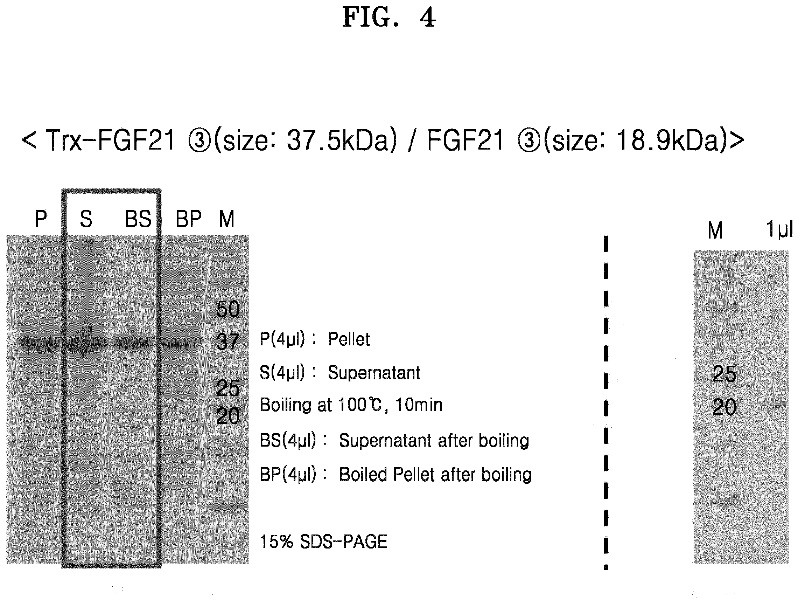

Specifically, a protein that is easy to express and is heat-resistant, such as Thioredoxin (Trx) or DnaK, was used as a tag, and *Escherichia coli* overexpressing Trx-FGF21, which is a Trx fused to an FGF21 variant, was disrupted by sonication and then centrifuged. The supernatant solution including Trx-FGF21 was incubated at high temperature (50 °C to 100 °C) for 10 minutes, and then the proteins denatured at high temperature and Trx-FGF21 were separated by centrifugation (FIG. 4, left).

In addition, to obtain a pure FGF21 variant with Trx removed, a cleavable linker must be inserted between the tag and the FGF21 variant. Specific sequences recognized by proteases such as Tobacco etch virus (TEV)-derived proteases or Thrombin and Factor Xa may be used as linkers. In addition, if a linker consisting of asparagine-glycine (Asn-Gly) is inserted between the tag and the FGF21 variant, the linker may be cleaved by heat-treatment (40 °C to 42 °C, 3 hours) using Hydroxylamine (NH₂OH). Therefore, FGF21 may be separated from the tag by protease, and then purified to a high purity of 90 % or above using two or fewer chromatographic techniques (FIG. 4, right).

### Example 3: Confirmation of activity of FGF21 variants

### 3.1: Confirmation of activity of the N-terminal truncation variant of FGF21

FGF21 (FGF21 △N4, 33-209aa), in which the signal peptide (1-28aa) and four N-terminus amino acids (29-32aa) of FGF21 were additionally removed, was produced through heat-treatment, and its activity was compared with commercially available FGF21 (29-209aa). FGF21 binds to the FGF receptor along with the co-receptor β-Klotho, and exerts various physiological effects by activating FGFR1c, one of the FGF receptors. Therefore, the FGF receptor activation by FGF21 was confirmed by using HEK293 cells with a reporter system to measure the degree of FGFR1c activation, that express both FGFR1c and β-Klotho.

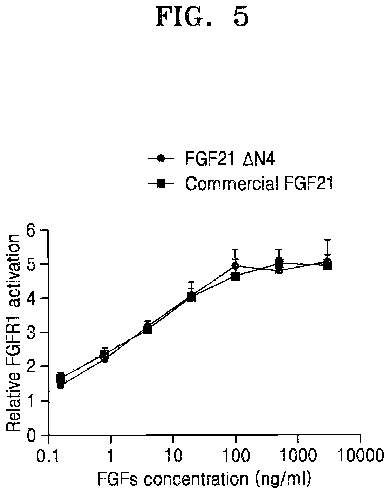

As a result, it may be confirmed that the activities of FGF21 ΔN4 purified through heat-treatment and commercial FGF21 were similar (FIG. 5). Indicating that even if 29^{th} to 33^{rd} amino acids are removed or produced through heat-treatment, the function of FGF21 to activate FGFR1c is not affected.

### 3.2: Confirmation of activity of FGF21 variants

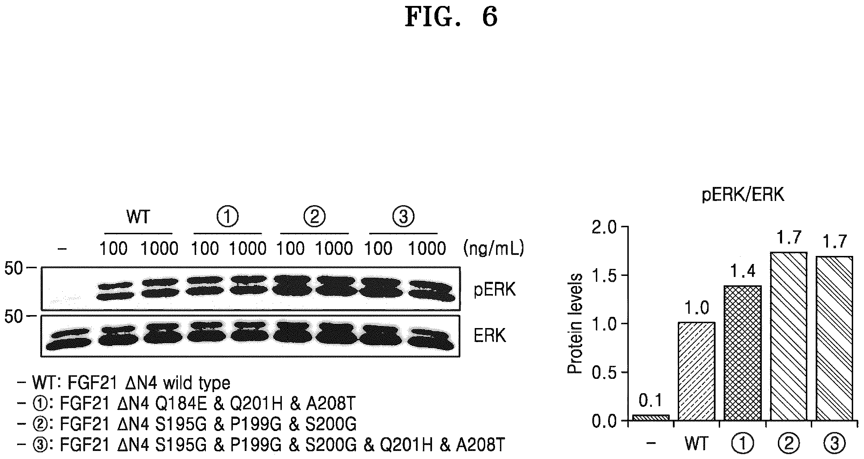

The effect of improving cell activity of FGF21 variants ①, ②, and ③ purified to high purity in Example 2 was measured through in vitro cell assay. First, using 3T3L1 adipocytes, FGFR signaling in adipocytes by FGF21 wild type and variants were confirmed by western blot. ERK is one of the representative signal transmitters in the intracellular signaling system in response to FGFR activation and becomes phosphorylated (pERK) upon FGFR activation. As a result of checking the level of pERK by FGF21, it was confirmed that the level increased about 1.4 to 1.7 times by the variants compared to the wild type, and confirmed that variant ② was more effective than variant ①. On the other hand, no significant difference was observed between variants ② and ③ (FIG. 6).

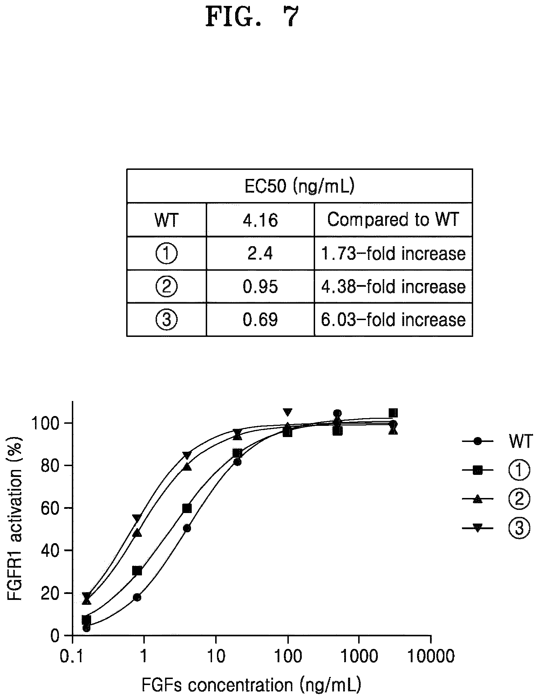

Next, the effect on the FGFR1 activation was confirmed depending on concentrations of the FGF21 wild type and the variants. This method uses HEK293 cells that express both FGFR1c and β-Klotho and are produced to express luciferase depending on the level of FGFR1c activation. This experiment was designed to more precisely determine the level of activation. As a result of analyzing the EC50 of each protein, it was confirmed that variants ①, ②, and ③ increased by 1.73-fold, 4.38-fold, and 6.03-fold, respectively, compared to the wild type (FIG. 7).

Based on the above experimental results, variant ③, a novel FGF21 variant developed in the present disclosure, has excellent activity and may be selected as an FGF21 biobetter.

The description of the present disclosure described above is for illustrative purposes, and those skilled in the art will understand that the present disclosure may be easily modified into other specific forms without changing its technical idea or essential features. Therefore, the embodiments described above should be understood in all respects as illustrative and not restrictive. For example, each component described in a single form may also be implemented in a distributed manner, and similarly, components described as distributed may also be implemented in a combined form.

The scope of the present application is indicated by the claims described below rather than the detailed description above, and all changes or modified forms derived from the meaning and scope of the claims and their equivalent concepts should be construed as being included in the scope of the present application.

## Claims

1. Polypeptides including FGF21 variants, wherein the variants are polypeptides containing one or more mutations compared to the amino acid sequence of the wild type FGF21.

2. The polypeptides of claim 1, wherein the mutations refer to one or more of a deletion, substitution, and insertion of an amino acid.

3. The polypeptide for the wild type FGF21 of claim 1, wherein the wild type FGF21 is a mature form in which a signal peptide is removed.

4. The polypeptide for the wild type FGF21 of claim 1, wherein the wild type FGF21 comprises an amino acid sequence of SEQ ID NO: 2.

5. The polypeptides for FGF21 variants of claim 1, wherein the FGF21 variants have deletions of 1 to 4 amino acids at the N-terminus.

6. The polypeptides for FGF21 variants of claim 1, wherein the FGF21 variants have one or more substitutions selected from the group consisting of Q184E, S195G, P199G, S200G, Q201H, and A208T.

7. The polypeptides for FGF21 variants of claim 1, wherein the FGF21 variants comprise a polypeptide containing S195G, P199G, S200G, Q201H, and A208T.

8. The polypeptides for FGF21 variants of claim 1, wherein the FGF21 variants comprise one of amino acid sequences of SEQ ID NOs: 3 to 6.

9. The polypeptides for FGF21 variants of claim 1, wherein the FGF21 variants have improved activities compared to the wild type FGF21.

10. A method of producing FGF21 variants, including:
a process of culturing a transgenic organism producing the FGF21 variants; and
a process of heating the transgenic organism, culture, or media at a temperature of 40 °C or above.

11. The method of producing the FGF21 variants of claim 10, wherein the FGF21 variants have heat-stability or heat-elasticity.

12. The method of producing the FGF21 variants of claim 10, wherein the FGF21 variants adopt the β-trefoil structure.

13. The method of producing the FGF21 variants of claim 10, wherein the FGF21 variants are additionally fused with a heat-stable protein tag.

14. The method of producing the FGF21 variants of claim 10, wherein the method additionally comprises a process of removing proteins denatured by heat-treatment.

15. A method of purifying FGF21 variants, comprising a process of heating a mixture containing the FGF21 variants and impurities at a temperature of 40 °C or above.

16. The method of purifying the FGF21 variants of claim 15, wherein the method additionally comprises a process of removing proteins denatured by heat-treatment.

17. A Pharmaceutical composition for treating metabolic disorders or cardiovascular disorders, comprising the FGF21 variants.
